# EUROPÄISCHE PATENTSCHRIFT

(11) **EP 3 908 132 B1**
(45) Veröffentlichungstag und Bekanntmachung des Hinweises auf die Patenterteilung: **21.05.2025**
(21) Anmeldenummer: 19836460.6
(22) Anmeldetag: 10.12.2019
(51) Int. Cl.: G01N 33/94, A61M 16/00, A61M 11/00, A61M 11/02, A61M 11/04, A61M 11/06, A61K 31/05, A61K 36/185, A24F 40/42, A61J 3/00, A61K 31/352, A61M 15/00, A61M 15/06

(54) **VERFAHREN ZUM QUALITATIVEN UND/ODER QUANTITATIVEN NACHWEIS VON IN EINER HANFPFLANZE ENTHALTENEN SUBSTANZEN UND KIT ZUR VERWENDUNG DARIN**
METHOD FOR QUALITATIVELY AND/OR QUANTITATIVELY DETECTING SUBSTANCES CONTAINED IN A HEMP PLANT AND KIT FOR USE THEREOF
PROCÉDÉ DE DÉTECTION QUALITATIVE ET/OU QUANTITATIVE DE SUBSTANCES CONTENUES DANS UNE PLANTE DE CHANVRE ET KIT POUR SON UTILISATION

(30) Priorität: 07.01.2019 DE 102019000016; 07.01.2019 DE 102019000018; 15.01.2019 DE 102019000199
(43) Veröffentlichungstag der Anmeldung: 17.11.2021
(73) Patentinhaber: LuxCan Innovation S.A., 1273 Luxembourg-Hamm (LU)
(72) Erfinder: SCHMITT, Fritz, 1855 Luxemburg (LU)
(74) Vertreter: Angerhausen, Christoph
(86) Internationale Anmeldenummer: PCT/DE2019/101064
(87) Internationale Veröffentlichungsnummer: WO 2020/143862

(56) Entgegenhaltungen:
- WO-A1-2012/154306
- WO-A1-2014/131114
- FR-A5- 2 239 174
- US-A- 3 715 189
- US-A- 4 104 027
- US-A- 4 196 167
- US-A1- 2015 017 732

## Beschreibung

Die vorliegende Erfindung betrifft ein Verfahren zum qualitativen und/oder quantitativen Nachweis von Substanzen, die in der Hanfpflanze enthalten sind. Die Erfindung betrifft ferner ein Kit zur Verwendung in diesem Verfahren.

### HINTERGRUND DER ERFINDUNG

Die WO 2012/154306 A1 beschreibt ein Kit, umfassend a) eine Ampulle; b) ein Material, dass eine Hanfpflanze oder Teile derselben umfasst; und c) einen Farbindikator, der geeignet ist, durch Inkontaktbringen mit der Hanfpflanze und/oder mindestens einem Teil davon unter Änderung der Farbe des Farbindikators zu reagieren, wobei das Material und der Farbindikator in der Ampulle angeordnet sind.

Die Legalisierung von Cannabis und die Verwendung als Medikament werfen viele Fragen der Qualitätssicherung durch den Produzenten und Vertreiber, insbesondere Apotheker und Ärzte, auf. Insbesondere muss durch den Apotheker gewährleistet und nach pharmazeutischen Regeln nachweisbar dokumentiert sein, welchen Wirkstoffgehalt ein in Verkehr gebrachtes Produkt hat.

Problematisch sind vor allem Angaben zum Gehalt von THC und CBD. Was den Gehalt dieser Leitsubstanzen betrifft, sind keine festen Ober- und Untergrenzen festgelegt, sollten aber bekannt sein bzw. bestimmt werden können. Der Gehalt einer Charge sollte in einem Bereich von +/- 10 Prozent des auf der Packung deklarierten Gehalts liegen. Das ist der Tatsache geschuldet, dass sich die einzelnen Sorten zum Teil erheblich hinsichtlich ihres Gehalts an Tetrahydrocannabinol (THC) und Cannabidiol (CBD) unterscheiden. Dass es auch innerhalb einer Sorte von Ernte zu Ernte erhebliche Unterschiede geben kann, findet dabei keine Berücksichtigung. Aus pharmazeutischer Sicht ist das unbefriedigend, insbesondere vor dem Hintergrund, dass zukünftig veränderte und strenge Auflagen zu erwarten sind.

In EP 1 32 313 A2 werden Cannabinoid-Nachweis-Methoden für Drogentestpackungen auf Basis von Diazonium-Reagenzien beschrieben. Die Reaktion wird auf einem Filterpapier durchgeführt und die Reagenzien hierbei in flüssiger Form oder als Spray aufgetragen. Diese und ähnliche Verfahren sind relativ umständlich durchzuführen. Zudem ist eine Aufbereitung des Probenmaterials durch Maßnahmen wie Trocknung, Extraktion, Filtration und Eindampfen, notwendig, um die entsprechenden Bestimmungen durchführen zu können. Dies erfordert einen erheblichen Arbeits- und Zeitaufwand und kann mitunter auch zu einer Veränderung der stofflichen Zusammensetzung des Probenmaterials führen. Außerdem ist bei diesen Methoden häufig die Verwendung von giftigen oder stark ätzenden Chemikalien erforderlich, was für eine breite Anwendung durch den Laien problematisch ist.

Es ist demzufolge die Aufgabe der vorliegenden Erfindung, ein Verfahren sowie das zugehörige Kit zum qualitativen und/oder quantitativen Nachweis von in Hanfpflanzen enthaltenen Substanzen bereitzustellen, das Nachteile des Stands der Technik überwindet, insbesondere ein Verfahren sowie das zugehörige Kit bereitzustellen, das die einfache und kostengünstige Analyse von Substanzen, die in Hanfpflanzen oder anderen hanfbasierten Produkten enthalten sein können, erlaubt, insbesondere durch Personen, die ungeübt in der chemischen Analyse sind, etwa Fachpersonal, Apotheker, Ärzte, Klinikpersonal etc. Das erfindungsgemäße Verfahren sowie das zugehörige Kit soll es insbesondere auch analytisch unerfahrenen Personen, etwa Apothekern, erlauben, den Wirkstoffgehalt in hanfbasierten Produkten mit einer Genauigkeit zu bestimmen, die den gängigen Auflagen genügt.

### BESCHREIBUNG DER ERFINDUNG

Diese Aufgabe wird gelöst durch ein Kit zum qualitativen und/oder quantitativen Nachweis einer oder mehrerer in der Hanfpflanze enthaltenen Substanz(en) umfassend: a) eine Ampulle, die transparent und evakuiert ist sowie zumindest teilweise aus Borosilikatglas besteht; b) ein Material, dass eine Hanfpflanze oder Teile derselben umfasst; und c) einen Farbindikator, der geeignet ist, durch Inkontaktbringen mit der Hanfpflanze und/oder mindestens einem Teil davon unter Änderung der Farbe des Farbindikators zu reagieren, wobei das Material und der Farbindikator in der luftdichten und evakuierten Ampulle angeordnet sind.

Das erfindungsgemäße Verfahren erlaubt eine einfache qualitative und quantitative Qualitätsprüfung von Hanf- bzw. Cannabis-basierten Materialien vor dem Inverkehrbringen. Diese Prüfung kann in einfacher und sicherer Weise auch durch die ungeübte Person, etwa einen Apotheker, vorgenommen werden, der dann die Qualität des von ihm vertriebenen Produkts gewährleisten und zertifizieren kann.

Das Material umfasst eine Hanfpflanze oder einen Teil derselben. Als ein Teil der Hanfpflanze kann auch eine einzelne aus der Hanfpflanze isolierte chemische Verbindung, vorzugsweise eine pharmazeutisch aktive Verbindung, verstanden werden.

Cannabis (Hanf) gehört zusammen mit der Gattung Humulus (Hopfen) der Familie der Cannabisaceae an, wobei jedoch Humulus keine Cannabinoide enthält. Innerhalb der Gattung Cannabis erfolgt eine botanische und chemotaxonomische Differenzierung und zwar in den Arten Cannabis sativa Linnaeus, Cannabis indica LAM und Cannabis ruderalis oder in die "Sammelart" Cannabis sativa L., bestehend aus den Unterarten Cannabis sativa ssp. sativa und ssp. indica. Darüber hinaus wird Cannabis in einen Drogen- und Faserhanf unterschieden, wobei die Unterscheidung aufgrund des mengenmäßigen Verhältnisses der Hauptcannabinoide Cannabidiol (CBD) und Δ⁹-Tetrahydrocannabinol (Δ⁹-THC) erfolgt (INN: Dronabinol). Faserhanf (auch: Nutzhanf, industrial hemp) wird hauptsächlich zur industriellen Fasergewinnung herangezogen und darf maximal ein Δ⁹-THC-Gehalt von 0,2 % aufweisen (z.B. Deutschland u.a.), während der Drogentyp ein Δ⁹-THC-Gehalt von ca. 5-15 % aufweisen kann (Marihuana, Haschisch). Cannabis sativa L. enthält über 400 verschiedene Inhaltsstoffe, davon gehören mehr als 60 Verbindungen der Klasse der Cannabinoide an. Die wichtigsten Cannabinoide sind im Folgenden dargestellt:
∘ *Cannabigerol-artige (CBG)* : Cannabigerol ((E)-CBG-C₅), Cannabigerol Monomethylether ((E)-CBGM-C₅ A), Cannabinerolsäure A ((Z)-CBGA-C₅ A), Cannabigerovarin ((E)-CBGV-C₃), Cannabigerolsäure A ((E)-CBGA-C₅ A), Cannabigerolsäure A Monomethylether ((E)-CBGAM-C₅ A), Cannabigerovarinsäure A ((E)-CBGVA-C₃ A) ;
∘ *Cannabichromen-artige (CBC)* : Cannabichromen (CBC-C₅), Cannabichromensäure A (CBCA-C₅ A), Cannabichromevarin (CBCV-C₃), Cannabichromevarinsäure A (CBCVA-C3 A);
∘ *Cannabidiol-artige (CBD):* Cannabidiol (CBD-C₅), Cannabidiol Monomethylether (CBDM-C₅), Cannabidiol-C4 (CBD-C₄), Cannabidivarin (CBDV-C₃), Cannabidiorcol (CBD-C₁), Cannabidiolsäure (CBDA-C₅), Cannabidivarinsäure (CBDVA-C₃);
∘ *Cannabinodiol-artige (CBND)* : Cannabinodiol (CBND-C₅), Cannabinodivarin (CBND-C₃);
∘ *Tetrahydrocannabinol-artige (THC):* Δ9-Tetrahydrocannabinol (Δ9-THC-C₅), Δ9-Tetrahydrocannabinol-C4 (Δ9-THC-C₄), Δ9-Tetrahydrocannabivarin (Δ9-THCV-C₃), Δ9-Tetrahydrocannabiorcol (Δ9-THCO-C₁), Δ9-Tetrahydrocannabinolsäure (Δ9-THCA-C₅ A), Δ9-Tetrahydrocannabinolsäure B (Δ9-THCA-C₅ B), Δ9-Tetrahydrocannabinolsäure-C4 (Δ9-THCA-C₄ A und/oder B), Δ9-Tetrahydrocannabivarinsäure A (Δ9-THCVA-C₃ A), Δ9-Tetrahydrocannabiorcolsäure (Δ9-THCOA-C₁ A und/oder B), (-)-Δ8-trans-(6aR,10aR)-Δ8-Tetrahydrocannabinol (Δ8-THC-C₅),(-)-Δ8-trans-(6aR,10aR)-Tetrahydrocannabinolsäure A (Δ8-THCA-C₅ A); (-)-(6aS,10aR)-Δ9-Tetrahydrocannabinol ((-)-cis-Δ9-THC-C₅);
∘ *Cannabinol-artige (CBN):* Cannabinol CBN-C₅, Cannabinol-C4 (CBN-C₄), Cannabivarin (CBN-C₃), Cannabinol-C2 (CBN-C₂), Cannabiorcol (CBN-C₁), Cannabinolsäure A (CBNA-C₅ A), Cannabinolmethylether (CBNM-C₅)
∘ *Cannabitriol-artige (CBT)* : (-)-(9R,10R)-trans-Cannabitriol ((-)-trans-CBT-C₅), (+)-(9S,10S)-Cannabitriol ((+)-trans-CBT-C₅), (±)-(9R,10S/9S,10R)-Cannabitriol ((+)-cis-CBT-C₅), (-)-(9R,10R)-trans[10-o-Ethyl-cannabitriol] ((-)-trans-CBT-OEt-C₅), (±)-(9R,10R/9S,1oS)-Cannabitriol-C3 ((±)-trans-CBT-C₃),8,9-Dihydroxy-Δ6a(10a) tetrahydrocannabinol (8,9-Di-OH-CBT-C₅), Cannabidiolsäure A (CBDA-C₅ 9-OH-CBT-C5 ester), (-)-(6aR,9S,10S,10aR)-9,10-Dihydroxy-hexahydrocannabinol, Cannabiripsol Cannabiripsol-C5, (-)-6a,7,10a-Trihydroxy-Δ9-tetrahydrocannabinol ((-)-Cannabitetrol), 10-Oxo-Δ6a(10a) tetrahydrocannabinol (OTHC);
∘ *Cannabielsoin-artige (CBE) :* (5aS, 6S, 9R, 9aR) - C₅-Cannabielsoin (CBE-C₅), (5aS,6S,9R,9aR)-C₃-Cannabielsoin (CBE-C₃), (5aS,6S,9R,9aR)-Cannabielsoinsäure A (CBEA-C₅ A), (5aS,6S,9R,9aR)-Cannabielsoinsäure B (CBEA-C₅ B), (5aS,6S,9R,9aR)-C3-Cannabielsoinsäure B (CBEA-C₃ B), Cannabiglendol-C3 (OH-iso-HHCV-C₃), Dehydrocannabifuran (DCBF-C₅), Cannabifuran (CBF-C₅) ;
∘ *Isocannabinoide:* (-)-Δ7-trans-(1R,3R,6R)-Isotetrahydrocannabinol, (±)-Δ7-1,2-cis-(1R,3R,6S/1S,3S,6R)-Isotetrahydrocannabivarin, (-)-Δ7-trans-(1R,3R,6R)-Isotetrahydrocannabivarin;
∘ *Cannabicyclol-artige (CBL)* : (±)-(1aS,3aR,8bR,8cR-Cannabicyclol (CBL-C₅), (±)-(1aS,3aR,8bR,8cR-Cannabicyclolsäure A (CBLA-C₅ A), (±)-(1aS,3aR,8bR,8cR-Cannabicyclovarin (CBLV-C₃);
∘ *Cannabicitran-artige (CBT)* : Cannabicitran (CBT-C₅) ;
*∘ Cannabichromanon-artige (CBCN)* : Cannabichromanon (CBCN-C₅), Cannabichromanon-C3 (CBCN-C₃), Cannabicoumaronon (CBCON-C₅).

Neben den o. g. erwähnten Cannabinoiden finden sich deren zugehörige Carbonsäuren in der Rohdroge. Diese Carbonsäuren sind biosynthetische Precursors.

Cannabiszubereitungen üben eine Vielzahl therapeutischer Wirkungen aus, darunter antispastische, analgetische, antiemetische, neuroprotektive, antiinflammatorische sowie Wirkungen bei psychiatrischen Erkrankungen (Grotenhermen F, Müller-Vahl K: The therapeutic potential of cannabis and cannabinoids.

In Deutschland ist seit 2011 ein Cannabisextrakt, der THC (Dronabinol) und CBD im Verhältnis 1:1 enthält (Nabiximols), für die Behandlung der mittelschweren bis schweren, therapieresistenten Spastik bei Multipler Sklerose (MS) als Sublingualspray (Sativex) arzneimittelrechtlich zugelassen.

Cannabidiol (CBD, CBD-C₅) ist das wichtigste nicht-psychotrope Cannabinoid der Gattung Cannabis und CBD ist kein Cannabinoidrezeptoragonist.

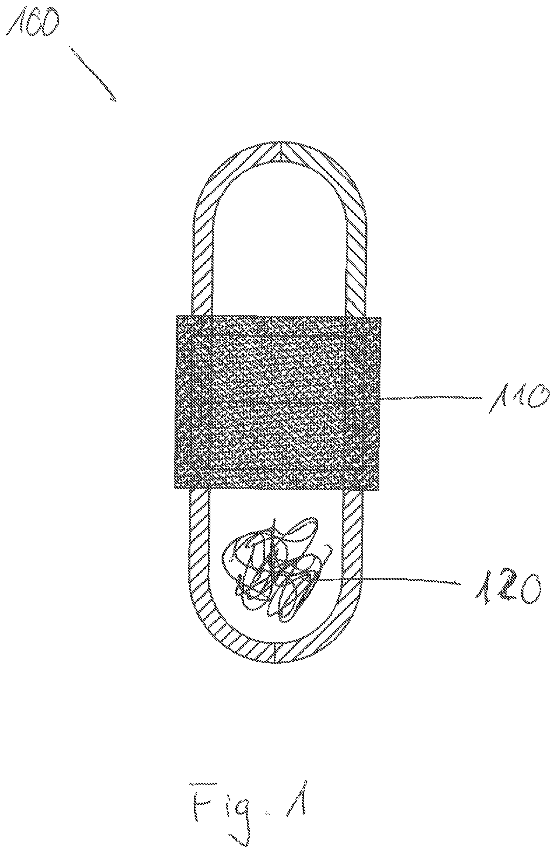

### Abb. 1: CBD (Strukturformel)

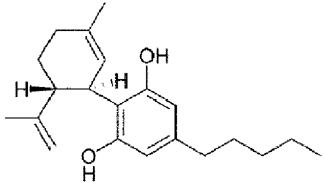

CBD kann synthetisch hergestellt werden (Michoulam R, Shvo Y., Hashish. I. The structure of cannabidiol, Tetrahedron. 1963, 19(12), 2073).

In einer Ausführungsform kann vorgesehen sein, dass das Material eine Cannabisblüte, Marihuana, Haschisch, Haschischöl, zumindest ein Cannabinoid oder eine Mischung davon umfasst.

In den verschiedenen Ausführungsformen kann das erfindungsgemäße Kit (sowie das unten beschriebene erfindungsgemäße Verfahren) zur Identifizierung und Qualitätsbestimmung von Cannabispräparaten wie Marihuana, Haschisch, Haschischöl und sonstigen Cannabinoid-haltigen Materialien, zur Bestimmung des Reifegrades von Hanfpflanzen, sowie zu Unterscheidung von Drogen- und auch bei jungen Hanfpflanzen in frischer oder auch getrockneter Form dienen.

Insbesondere kann vorgesehen sein, dass das erfindungsgemäße Kit/Verfahren zum chemischen Nachweis verschiedener phenolartiger Verbindungen, ätherischen Ölen, Resinoiden, Frischpflanzen, Pflanzendrogen, Pflanzenextrakten- und Auszügen und Geruchstoffen, sowie zur deren Identifizierung und Charakterisierung und Qualitätsbestimmung dient.

In einer weiteren Ausführungsform kann vorgesehen sein, dass der Farbindikator eine farbbildende Substanz umfasst, die vorzugsweise ausgewählt ist aus der Gruppe, bestehend aus Echtschwarz, Echtblausalz, Dibromchinonchlorimid, Dichlorchinonchlorimid, Vanillin, Salicylaldehyd, Formaldehyd, Acetaldehyd, p-Dimethylaminobenzaldehyd, Diethylaminobenzaldehyd, Eisen-III-Chlorid, Aminophenol, Aminoantipyrin, Kaliumhexacyanoferrat und einem Gemisch von zwei oder mehreren davon.

Ebenso kann vorgesehen sein, dass der Farbindikator zumindest ein Lösungsmittel umfasst, dass vorzugsweise ausgewählt ist aus der Gruppe bestehend aus Wasser und ein- oder mehrwertigen Alkoholen.

Insbesondere kann vorgesehen sein, dass als Lösungsmittel ein, oder mehrwertige Alkohole alleine oder deren Gemische verwendet, welche einerseits eine optimale Extraktion der Wirkstoffe aus dem Material und andererseits aber auch gute Löseeigenschaften für die verwendete farbbildende Substanz sowie gegebenenfalls weitere Bestandteile des Farbindikator bereitstellt und eine störungsfreie Farbreaktion garantieren.

In einer weiteren Ausführungsform kann vorgesehen sein, dass der Farbindikator ferner ein Reagenz umfasst, dass das Reagieren von dem Material mit der farbbildenden Substanz unterstützt, wobei das Reagenz vorzugsweise eine basische Verbindung ist, besonders bevorzugt ausgewählt ist aus der Gruppe, bestehend aus Alkalihydroxid, Alkalicarbonat, Ammonium- oder Alkalisalzen einer organischen Säure und einem Gemisch von zwei oder mehr davon.

Ferner kann vorgesehen sein, dass der Farbindikator ferner ein Trägermaterial umfasst, vorzugsweise ein saugfähiges neutrales Trägermaterial. Das Trägermaterial kann offenporig oder geschlossenporig sein. Die einzelnen Bestandteile des Farbindikator, etwa die farbgebende Substanz oder das Reagenz, können in den Poren enthalten sein.

In einer Ausführungsform kann vorgesehen sein, dass der Farbindikator aus einer ersten Lösung der farbbildenden Substanz in Wasser oder/und primären, sekundären und/oder tertiären Alkoholen und einer zweiten Lösung einer Base, beispielsweise Alkalihydroxid, in Wasser oder Alkoholen oder deren Gemischen zusammengesetzt ist.

Erfindungsgemäß ist vorgesehen, dass die in dem Farbindikator enthaltenen Bestandteile alleine oder zusammen mit einer Änderung der Farbe des Farbindikator auf die Anwesenheit von Substanzen reagieren, die in dem erfindungsgemäßen Material enthalten sind.

In einer weiteren Ausführungsform kann vorgesehen sein, dass die Ampulle eine teilbare Ampulle ist, die aus zwei oder mehreren Teilen besteht, die miteinander verbunden werden können, um die Ampulle zu bilden, wobei der Farbindikator auf zumindest einem Teil einer Innenwand eines der Teile, die miteinander verbunden werden können, um die Ampulle zu bilden, angeordnet ist.

Erfindungsgemäß ist es vorgesehen, dass die Ampulle luftdicht und/oder luftdicht verschließbar ist.

Beispielsweise kann vorgesehen sein, dass die Ampulle aus drei verschiedenen Teilen besteht, die luftdicht miteinander verbunden sind. Die Verbindung der einzelnen Teile der Ampulle kann auf verschiedene Weise realisiert werden, etwa durch Verschrauben der Teile miteinander. Es kann vorgesehen sein, dass auf einer Innenwand (oder eines Teils derselben) eines Teils (oder mehrerer Teile) der teilbaren Ampulle der Farbindikator angeordnet ist. Die Innenwand ist hierbei der Teil, der nach Zusammensetzen und Verbinden der Teile zum Innern der Ampulle hin angeordnet ist. Hierbei kann vorgesehen sein, dass der Träger auf der Innenwand der Ampulle oder eines Teils derselben angeordnet ist, etwa durch eine Klebeverbindung zwischen dem Träger und der Innenwand. Auf dem Träger, zum Innern der Ampulle hin, sind dann die übrigen Bestandteile des Farbindikator, insbesondere die farbbildende Substanz angeordnet, um mit dem Material oder aus diesem Material freigesetzten Bestandteilen, etwa mit aus der Hanfpflanze freigesetzten Substanzen, in Kontakt gebracht zu werden.

In einer Ausführungsform kann vorgesehen sein, dass der Teil, auf dessen Innenwand der Farbindikator zumindest teilweise angeordnet ist, eine Distanzscheibe ist, die durch Verschrauben mit einem oder mehreren weiteren Teilen der teilbaren Ampulle verbunden werden kann.

In einer Ausführungsform kann vorgesehen sein, dass die einzelnen Bestandteile der teilbaren Ampulle scheibenförmige verschraubbare Ringe umfassen, die beidseitig miteinander verbunden werden können.

In einer weiteren Ausführungsform kann vorgesehen sein, dass die Ampulle, insbesondere das Innere der Ampulle, korrosionsfest ist, insbesondere gegenüber Säuren und Basen.

Da die Ampulle transparent ist und zumindest teilweise aus Borosilikatglas besteht, wird erreicht, dass der Farbumschlag des Farbindikator optisch einfach ausgewertet werden kann, gegebenenfalls zusätzlich entsprechend gespeichert und mit in einer Datenbank als Referenz hinterlegten Daten verglichen werden kann.

Erfindungsgemäß ist es vorgesehen, dass die Ampulle evakuiert ist. Die Aufgabe wird ferner gelöst durch ein Verfahren zum qualitativen und/oder quantitativen Nachweis einer oder mehrerer in der Hanfpflanze enthaltenen Substanz(en), umfassend die Schritte: a) Bereitstellen des erfindungsgemäßen Kits; b) Inkontaktbringen des Materials oder von Teilen davon mit dem Farbindikator; und c) Detektieren einer Änderung der Farbe des Farbindikators. Das Inkontaktbringen des Materials oder von Teilen davon mit dem Farbindikator umfasst das Erhitzen des Materials oder von Teilen davon in der transparenten, evakuierten sowie zumindest teilweise aus Borosilikatglas bestehenden Ampulle.

Es ist vorgesehen, dass das Inkontaktbringen des Materials oder von Teilen davon mit dem Farbindikator das Erhitzen des Materials oder von Teilen davon in der Ampulle umfasst. In diesem Zusammenhang kann vorgesehen sein, dass das Erhitzen induktives Erhitzen umfasst.

In einer weiteren Ausführungsform kann vorgesehen sein, dass das Detektieren unter Verwendung einer Farbvergleichsskala, eines optischen Sensors, eines chemischen Sensors oder zwei oder mehr davon erfolgt.

Ebenso kann vorgesehen sein, dass das Verfahren ferner einen Schritt, nach dem Detektieren, umfasst, umfassend das Abgleichen von Informationen, die durch das Detektieren erhalten werden, mit in einer Datenbank hinterlegten Daten.

### DETAILLIERTE BESCHREIBUNG DER ERFINDUNG

Im Folgenden soll die Erfindung unter Bezugnahme auf die Zeichnungen anhand konkreter Ausführungsformen beschrieben werden. Hierbei ist zu verstehen, dass die Bezugnahme auf die konkreten Ausführungsformen lediglich der Veranschaulichung der Erfindung dient. Die Merkmale der konkreten Ausführungsformen sind nicht notwendigerweise limitierend für die Erfindung, können jedoch, insbesondere in Kombination mit den im vorangehenden genannten Ausführungsformen, zur vorteilhaften Verwirklichung der Erfindung beitragen.
Fig.1: schematische Darstellung einer Ampulle gemäß einer Ausführungsform der Erfindung;
Fig.2: schematische Darstellung einer Ampulle gemäß einer weiteren Ausführungsform der Erfindung; und
Fig.3: schematische Darstellung einer Ampulle gemäß einer weiteren Ausführungsform der Erfindung.

Beschrieben ist ein Verfahren zur Qualitätsprüfung von pharmazeutischem Cannabis, bei dem eine Ampulle in der sich ein Material, etwa Cannabisblüten oder Teile davon, befindet so gestaltet werden kann, dass die Ampulle selbst oder durch Kombinieren mit zusätzlichen Elementen die entsprechenden Prüfungen für das Inverkehrbringen durch den Apotheker gewährleistet und Prüfzertifikate nach den pharmazeutischen Regeln nachweisbar dokumentiert und erstellt werden können.

Erfindungsgemäß ist vorgesehen, dass die Cannabisblüten oder Teile davon sofort nach der Ernte hermetisch verpackt werden und in einer speziellen transparenten Ampulle aus Borosilikatglas so verschlossen werden, dass nur durch Zerstören der Ampulle der Wirkstoff entnommen werden kann. Innerhalb der evakuierten Ampulle kann mit verschiedenen hierin beschriebenen Hilfsmitteln eine chemisch-analytische Untersuchung erfolgen. Die Ergebnisse können als Daten auf entsprechende Strichcodes o.Ä. angebracht werden. Weitere entsprechende relevante Informationen sind: z.B. Anbau, Ernte, Verarbeitung, Qualitätsprüfung, Lagerung, Verpackung, sind natürlich auch manipulationssicher vorhanden. Das kann dadurch erreicht werden, dass ein Lasercode in die Oberfläche eingebrannt wird. Die Daten können in einer Dockingstation ausgelesen werden.

Mit dem neuen Verfahren, das in den Ansprüchen näher beschrieben wird, ist es möglich, sogar während des Wachstums der Pflanze Qualitätsprüfungen durchzuführen. Denkbar ist, dass die Cannabisblüte in der Ampulle während des Wachstums untersucht wird.

Chemische Untersuchungen finden in der Ampulle statt. Die Ampulle ist so gestaltet, dass die Prüfelemente mit entsprechenden Reagenzien ein Teil der Ampulle darstellen oder an der Ampulle angeschraubt werden können.

Durch das in den Ansprüchen beschriebene Kit ist das Einhalten der üblichen Anforderungen gewährleistet, insbesondere:
- gleichbleibende Produktqualität
- enthaltene Substanzen
- Angaben der Konzentration der Wirkstoffe
- Vermeidung jeglicher Art von Kontamination
- Rückverfolgbarkeit

Das erfindungsgemäße Kit und das erfindungsgemäße Verfahren ermöglichen Fachpersonal, Apothekern, Ärzten oder Klinikpersonal den chemischen Nachweis von phenolartigen Substanzen, insbesondere von Cannabinoiden und solche Stoffe enthaltenden Materialien.

Die Probe kann direkt, d. h. beispielsweise in vorbehandelter oder unbehandelter Form, auch ohne vorherige Trocknung direkt mit einem farbbildenden Reagenz versetzt werden. Dies kann dadurch erreicht werden, dass eine wie in der Figur 1 gezeigte mehrteilige Ampulle 100 einen ringförmigen Adapter 110 aufschraubbar umfasst. Der ringförmige Adapter 110 enthält in seiner den Ring ausfüllenden Fläche den Farbindikator, der für die Analyse des Materials 120 nötig ist.

Der Farbindikator kann als Lösung einer farbbildenden Substanz und des Reagenz mit oder ohne Zusatz organischer Lösungsmittel eingesetzt werden.

Die farbbildende Substanz wird vorzugsweise in Form von verdünnten Lösungen der farbbildenden Substanz in Wasser und/oder primären, sekundären und tertiären Alkoholen mit oder ohne Zusatz von organischen Lösungsmittel wie gesättigten und ungesättigten Kohlenwasserstoffen, halogenierten Kohlenwasserstoffen, Ethern, Ketonen, Carbonsäure Estern und/oder aromatischen Kohlenwasserstoffen vorgelegt.

Das Reagenz ist vorzugsweise eine Base, wie etwa ein Alkalihydroxid und/oder ein Alkalicarbonat, ein ggf. durch einen oder mehrere organische Reste, z. B. AlkylGruppen, substituiertes Ammonium- oder Alkalisalz einer organischen Säure oder Gemischen davon und kann in Form einer verdünnten Lösung zusammen mit der farbbildenden Substanz in Wasser und/oder primären, sekundären und Alkoholen oder deren Gemischen vorliegen. Gegebenenfalls können Ammonium- oder Alkalisalze organischer Säuren wie Essigsäure, Propionsäure, Buttersäure, Äpfelsäure, Sorbinsäure, Fumarsäure, Benzoesäure, Phenylessigsäure, Phthalsäure, Naphthylessigsäure oder Mischungen daraus verwendet werden.

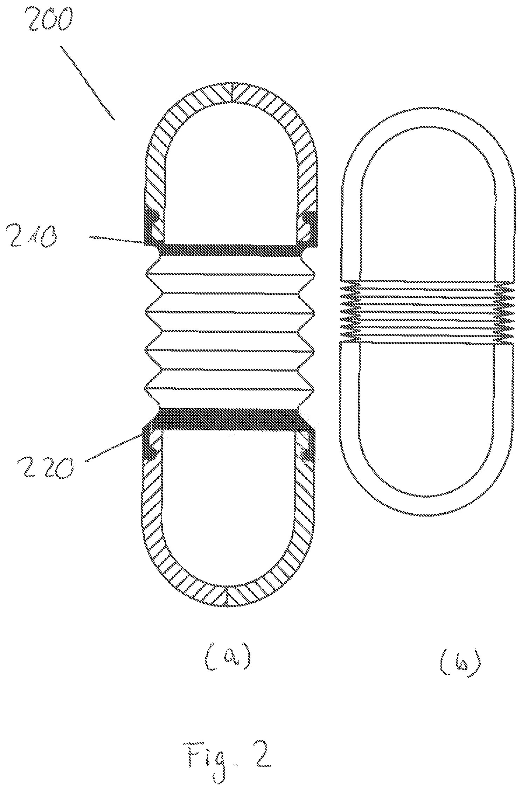

Überraschenderweise ist es gelungen, ein Verfahren zu entwickeln, in dem speziell ausgewählte Farbreaktionen so modifiziert werden, dass die für die farbreaktionsrelevanten Bestandteile unmittelbar dem als Extraktionsmittel fungierenden Lösungsmittel zugefügt und in der chemischen Nachweisreaktion direkt eingesetzt werden. In der in der Figur 2 gezeigten Ampulle 200 wird dies dadurch erreicht, dass das Testmaterial (nicht gezeigt), in diesem Fall Cannabisblüten, in der Ampulle 200 zwischen einem ersten Distanzring 210 und einem zweiten Distanzring 220 eingepresst wird. In der Figur 2a wird die Ampulle im nicht-zusammengepressten Zustand gezeigt, wohingegen die Figur 2b die gleiche Ampulle im zusammengepressten Zustand zeigt.

Der Farbindikator und dessen Farbveränderung können mit entsprechenden optischen und chemischen Sensoren ausgewertet werden.

Gemäß dem Öffnungsmessverfahren wird erreicht, dass der vorherrschende Phenolkörper durch Entwicklung eines spezifischen, deutlich unterscheidbaren Farbtones einzeln für das Auge sichtbar gemacht und so auf bisher nicht bekannte, einfache und schnelle Weise direkt nachgewiesen werden kann. Dadurch, dass das angewendete Verfahren innerhalb der Ampulle stattfindet, sind äußere Einflüsse ausgeschlossen. Durch Verfeinerung des Verfahrens unter zur Hilfenahme von elektronischen optischen Sensoren ist eine Datenerhebung und Speicherung der Daten möglich. Somit ist eine zusätzlich optimale, besonders leicht ablesbare Abstufung, sowie eine Dauerhafte anhaltende Speicherung der entstandenen Farbtöne erreicht. Auf diese Weise ist es erstmals auch möglich, frisches, unbehandeltes Probematerial und sogar Frischpflanzen in vorbehandelter oder unbehandelter Form auch ohne vorherige Trocknung, z. B. am Feld, direkt der Untersuchung zu unterwerfen und nach phenolischen oder cannabinoiden Bestandteilen zu analysieren. Ein Vergleich der aufgezeichneten Daten im Laufe des Wachstums ist hierdurch möglich. Besonders bewährt hat sich das erfindungsgemäße Verfahren beim Nachweis von Cannabinoiden. Damit ist erstmals eine Unterscheidung von Drogen- und Industriehanf bei jungen oder erwachsenen, männlichen oder weiblichen Pflanzen ab einem Alter von zwei Wochen möglich. Des Weiteren kann die Bestimmung des Reifegrades von Hanfpflanzen auf schnelle und einfache Weise sogar direkt an Frischpflanzen vorgenommen werden. Die Einsatzbereiche des erfindungsgemäßen Verfahrens umfassen die Cannabis-Forschung und Medizin, den Cannabis-Konsum (Qualitätskontrolle) und behördliche Anforderung an den Apotheker, Arzt usw. Weiterhin kann das Verfahren zum Nachweis von Cannabis-Präparaten in biologischen Probenmaterialien verwendet werden. Durch spezielle Modifikationen der Reagenzien und der Verfahren konnten neue Methoden zum selektiven Nachweis einzelner spezifischer Cannabinoide gefunden werden.

Aufgrund der einfachen Konzeption des Verfahrens und der dazu nötigen Reagenzien eignet sich dieses Verfahren besonders für Analysenpackungen die direkt an Ort und Stelle eingesetzt werden können. Mittels Softwaregestützter, photometrischer Messung der Absorptionshauptmaxima der entwickelten Farbtöne, können die Verfahren auch zur quantitativen Bestimmung der nachgewiesenen Phenolkörper herangezogen werden. Eine Datenspeicherung und Vergleichsmöglichkeiten erweitert die Sicherheit im Umgang des medizinischen Cannabis.

In der Reagenzlösung werden als Lösungsmittel vorzugsweise ein- oder mehrwertige Alkohole alleine oder als Gemisch verwendet, welche einerseits eine optimale Extraktion der Wirkstoffe und andererseits aber auch gute Löseeigenschaften für die verwendeten Bestandteile des Verbändekartons, die für die Verbindung verantwortlich sind, und eine störungsfreie Farbreaktion garantieren. Die zur Farbentwicklung notwendigen Bestandteile des Farbendiktators wurden so konzipiert, dass sie zwecks möglichst einfacher und effektiver Handhabung nur in Tropfenmengen dem Reagenzgemisch zugegeben werden müssen. Durch die Zugabe von Salzen organischer Säuren zu der Reagenzlösung kann eine bessere Ablesbarkeit der entwickelten Farbtöne, sowie eine verbesserte Haltbarkeit derselben erzielt werden.

Bei entsprechender Abspeicherung der Farbtöne kann eine Datenbank erstellt werden, die eine Zuordnung der Hanfpflanzen nach Ort und Herkunft zulässt.

### Vergleichendes Beispiel:

### Beispiele von Farbdarstellungen:

- Hanf-Frischpflanzen unreifer Drogenhanf violett rötlich ausgereifter blaugrün
- Reifer EU-Industriehanf violettrötlich
- Thymol tiefblau
- Cannabidiol (CBD) violettrosa
- Tetrahydrocannabiol grünblau
- Cannabinol (CBN) blau
Für die Bestimmung des Reifegrades wird frisches, nicht getrocknetes Pflanzenmaterial verwendet, da beim Trocknen der Cannabinoid-Gehalt verändert werden kann (z. B. Umwandlung von Cannabidiol zu THC). Zur Unterscheidung von Industrie-oder Drogenhanf werden voll ausgebildete fingrige Normalblätter (frisch oder getrocknet) der Pflanze und nicht die Blüten-oder Fruchtstände verwendet. Durch einen Test der normalen, fingrigen Blätter einer bestimmten, mindestens zwei Wochen alten Jungpflanze kann deren maximal erreichbares zukünftiges Cannabinoid-Spektrum ermittelt werden. Wenn die entwickelten Farbtöne zu intensiv und damit schwer ablesbar sind, sollte der Test mit reduzierter Probenmenge wiederholt werden. Zu kleine Probenmengen können wiederum blasse, undeutliche und ins gelbe verschobene Farbtöne ergeben. In diesem Fall sollte der Test mit größerer Probenmenge wiederholt werden.

### Durchführung der Tests:

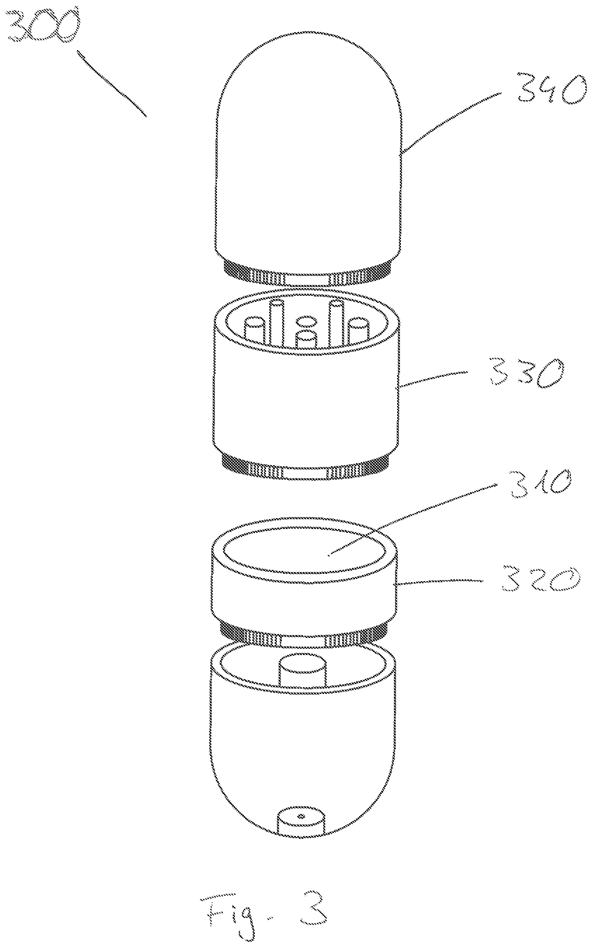

Eine kleine Menge der Proben wird auf eine ringförmige mit Reagenz getränkte Fläche 310 eines Teils der in Figur 3 gezeigten teilbaren Ampulle 300 gegeben. Ein Distanzstück 320 mit einer Lösung der farbgebenden Substanz wird mit einem Distanzstück 330 mit einer Lösung des Reagenzes mit einer Kunststoffkappe 340 verschlossen und innerhalb von etwa 1 min mehrere Male durchgeschüttelt. Innerhalb einer Minute kann die entstandene Färbung der überstehenden Lösung abgelesen werden. Die besten Ergebnisse werden bei durchscheinendem Tageslicht oder gegen eine helle Fläche sichtbar. Ein sehr genaues Ergebnis liefert natürlich eine softwaregesteuerte Auswertung unter Zuhilfenahme photospektrometrischer Vorrichtung.

Die in der vorangehenden Beschreibung und den beigefügten Ansprüchen offenbarten Eigenschaften können, separat oder in einer Kombination, Gegenstand zur Realisierung der Aspekte der in den unabhängigen Ansprüchen gemachten Offenbarung in unterschiedlichen Formen davon sein.

## Patentansprüche

1. Kit zum qualitativen und/oder quantitativen Nachweis einer oder mehrerer in der Hanfpflanze enthaltenen Substanz(en), umfassend:
a) eine Ampulle (100), die transparent und evakuiert ist sowie zumindest teilweise aus Borosilikatglas besteht;
b) ein Material (120), das eine Hanfpflanze oder Teile derselben umfasst; und
c) einen Farbindikator, der geeignet ist, durch Inkontaktbringen mit der Hanfpflanze und/oder mindestens einem Teil davon unter Änderung der Farbe des Farbindikators zu reagieren;
wobei das Material (120) und der Farbindikator in der evakuierten Ampulle (100) angeordnet sind.

2. Kit nach Anspruch 1, wobei das Material (120) eine Cannabisblüte, Marihuana, Haschisch, Haschischöl, zumindest ein Cannabinoid oder eine Mischung davon umfasst.

3. Kit nach Anspruch 1 oder 2, wobei der Farbindikator eine farbbildende Substanz umfasst, die vorzugsweise ausgewählt ist aus der Gruppe, bestehend aus Echtschwarz, Echtblausalz, Dibromchinonchlorimid, Dichlorchinonchlorimid, Vanillin, Salicylaldehyd, Formaldehyd, Acetaldehyd, p-Dimethylaminobenzaldehyd, Diethylaminobenzaldehyd, Eisen-III-Chlorid, Aminophenol, Kaliumhexacyanoferrat und einem Gemisch von zwei oder mehreren davon.

4. Kit nach einem der vorangehenden Ansprüche, wobei der Farbindikator zumindest ein Lösungsmittel umfasst, dass vorzugsweise ausgewählt ist aus der Gruppe bestehend aus Wasser und ein- oder mehrwertigen Alkoholen.

5. Kit nach einem der Ansprüche 3 oder 4, wobei der Farbindikator ferner ein Reagenz umfasst, dass das Reagieren des Materials mit der farbbildenden Substanz unterstützt, wobei das Reagenz vorzugsweise eine basische Verbindung ist, besonders bevorzugt ausgewählt ist aus der Gruppe, bestehend aus Alkalihydroxid, Alkalicarbonat, Ammonium- oder Alkalisalzen einer organischen Säure und einem Gemisch von zwei oder mehr davon.

6. Kit nach einem der Ansprüche 3 bis 5, wobei der Farbindikator ferner ein Trägermaterial umfasst, vorzugsweise ein saugfähiges neutrales Trägermaterial.

7. Kit nach einem der vorangehenden Ansprüche, wobei die Ampulle (100) eine teilbare Ampulle (300) ist, die aus zwei oder mehreren Teilen (310, 320, 330, 340) besteht, die miteinander verbunden werden können, um die Ampulle (300) zu bilden, wobei der Farbindikator auf zumindest einem Teil einer Innenwand eines der Teile, die miteinander verbunden werden können, um die Ampulle (300) zu bilden, angeordnet ist.

8. Kit nach Anspruch 7, wobei der Teil, auf dessen Innenwand der Farbindikator zumindest teilweise angeordnet ist, eine Distanzscheibe ist, die durch Verschrauben mit einem oder mehreren weiteren Teilen (310, 320, 330, 340) der teilbaren Ampulle (300) verbunden werden kann.

9. Verfahren zum qualitativen und/oder quantitativen Nachweis einer oder mehrerer in der Hanfpflanze enthaltenen Substanz(en), umfassend die Schritte:
a) Bereitstellen eines Kits nach einem der Ansprüche 1 bis 8;
b) Inkontaktbringen des Materials (120) oder von Teilen davon mit dem Farbindikator; und
c) Detektieren einer Änderung der Farbe des Farbindikators;
**dadurch gekennzeichnet, dass** das Inkontaktbringen des Materials (120) oder von Teilen davon mit dem Farbindikator das Erhitzen des Materials (120) oder von Teilen davon in der transparenten, evakuierten sowie zumindest teilweise aus Borosilikatglas bestehenden Ampulle (100) umfasst.

10. Verfahren nach Anspruch 9, wobei das Erhitzen induktives Erhitzen umfasst.

11. Verfahren nach einem der Ansprüche 9 oder 10, wobei das Detektieren unter Verwendung einer Farbvergleichsskala, eines optischen Sensors, eines chemischen Sensors oder zwei oder mehr davon erfolgt.

12. Verfahren nach einem der Ansprüche 9 bis 11, ferner umfassend einen Schritt, nach dem Detektieren, umfassend das Abgleichen von Informationen, die durch das Detektieren erhalten werden, mit in einer Datenbank hinterlegten Daten.

## Claims

1. A kit for the qualitative and/or quantitative detection of one or more substance(s) contained in the hemp plant, comprising:
a) an ampoule (100) which is transparent and evacuated and at least partially consists of borosilicate glass;
b) a material (120) comprising a hemp plant or parts thereof; and
c) a color indicator adapted to react by contacting the hemp plant and/or at least a part thereof while changing the color of the color indicator;
wherein the material (120) and the color indicator are disposed in the evacuated ampoule (100).

2. The kit of claim 1, wherein the material (120) comprises a cannabis blossom, marijuana, hashish, hashish oil, at least one cannabinoid, or a mixture thereof.

3. The kit of claim 1 or 2, wherein the color indicator comprises a color-forming substance, which is preferably selected from the group consisting of real black, real blue salt, dibromoquinone chlorimide, dichloroquinone chlorimide, vanillin, salicylaldehyde, formaldehyde, acetaldehyde, p-dimethylaminobenzaldehyde, diethylaminobenzaldehyde, ferric chloride, aminophenol, potassium hexacyanoferrate, and a mixture of two or more thereof.

4. The kit of any one of the preceding claims, wherein the color indicator comprises at least one solvent, which is preferably selected from the group consisting of water and mono- or polyhydric alcohols.

5. The kit of any one of claims 3 or 4, wherein the color indicator further comprises a reagent that assists the reaction of the material with the color-forming substance, wherein the reagent is preferably a basic compound, more preferably selected from the group consisting of alkali hydroxide, alkali carbonate, ammonium or alkali salts of an organic acid, and a mixture of two or more thereof.

6. The kit of any one of claims 3 to 5, wherein the color indicator further comprises a carrier material, preferably an absorbent neutral carrier material.

7. The kit of any one of the preceding claims, wherein the ampoule (100) is a separable ampoule (300) consisting of two or more parts (310, 320, 330, 340) that can be joined together to form the ampoule (300), wherein the color indicator is disposed on at least a part of an inner wall of one of the parts that can be joined together to form the ampoule (300).

8. The kit of claim 7, wherein the part on the inner wall of which the color indicator is at least partially disposed is a spacer which can be joined by screwing to one or more further parts (310, 320, 330, 340) of the separable ampoule (300).

9. A method for the qualitative and/or quantitative detection of one or more substance(s) contained in the hemp plant, comprising the steps of:
a) providing a kit according to any one of claims 1 to 8;
b) contacting the material (120) or parts thereof with the color indicator; and
c) detecting a change in the color of the color indicator;
**characterized in that** contacting the material (120) or parts thereof with the color indicator comprises heating the material (120) or parts thereof in the transparent, evacuated and at least partially borosilicate glass ampoule (100).

10. The method of claim 9, wherein the heating comprises inductive heating.

11. The method of any one of claims 9 or 10, wherein the detecting is performed using a color comparison scale, an optical sensor, a chemical sensor, or two or more thereof.

12. The method of any one of claims 9 to 11, further comprising a step, after the detecting, of matching information obtained by the detecting with data stored in a database.

## Revendications

1. Kit pour la détection qualitative et/ou quantitative d'une ou plusieurs substances contenues dans la plante de chanvre, comprenant :
a) une ampoule (100) qui est transparente et sous vide et qui est constituée au moins en partie de verre de borosilicate ;
b) un matériau (120) qui comprend une plante de chanvre ou des parties de celle-ci ; et
c) un indicateur coloré capable de réagir en changeant de couleur au contact de la plante de chanvre et/ou d'au moins une partie de celle-ci ;
le matériau (120) et l'indicateur coloré étant placés dans l'ampoule (100) sous vide.

2. Kit selon la revendication 1, dans lequel le matériau (120) comprend une fleur de cannabis, de la marijuana, du haschisch, de l'huile de haschisch, au moins un cannabinoïde ou un mélange de ces éléments.

3. Kit selon la revendication 1 ou 2, dans lequel l'indicateur coloré comprend une substance chromogène qui est de préférence choisie dans le groupe constitué par le noir véritable, le sel bleu véritable, le dibromochinone chloroimide, le dichlorochinone chloroimide, la vanilline, le salicylaldéhyde, le formaldéhyde, l'acétaldéhyde, le p-diméthylaminobenzaldéhyde, le diéthylaminobenzaldéhyde, le chlorure de fer, l'aminophénol, l'hexacyanoferrate de potassium et un mélange de deux ou plusieurs de ces composés.

4. Kit selon l'une des revendications précédentes, dans lequel l'indicateur coloré comprend au moins un solvant qui est de préférence choisi dans le groupe constitué par l'eau et les alcools mono- ou polyvalents.

5. Kit selon l'une des revendications 3 ou 4, dans lequel l'indicateur coloré comprend en outre un réactif qui favorise la réaction du matériau avec la substance chromogène, le réactif étant de préférence un composé basique, de préférence choisi dans le groupe constitué par un hydroxyde alcalin, un carbonate alcalin, des sels d'ammonium ou alcalins d'un acide organique et un mélange de deux ou plusieurs de ces éléments.

6. Kit selon l'une des revendications 3 à 5, dans lequel l'indicateur coloré comprend en outre un matériau de support, de préférence un matériau de support neutre absorbant.

7. Kit selon l'une des revendications précédentes, dans lequel l'ampoule (100) est une ampoule divisible (300) qui se compose de deux ou plusieurs parties (310, 320, 330, 340) qui peuvent être assemblées pour former l'ampoule (300), l'indicateur de couleur étant disposé sur au moins une partie d'une paroi intérieure de l'une des parties qui peuvent être assemblées pour former l'ampoule (300).

8. Kit selon la revendication 7, dans lequel la partie sur la paroi intérieure de laquelle l'indicateur coloré est au moins partiellement agencé est une rondelle d'écartement qui peut être reliée par vissage à une ou plusieurs autres parties (310, 320, 330, 340) de l'ampoule divisible (300).

9. Procédé de détection qualitative et/ou quantitative d'une ou plusieurs substances contenues dans la plante de chanvre, comprenant les étapes suivantes :
a) préparation d'un kit selon l'une des revendications 1 à 8 ;
b) mise en contact du matériau (120) ou de parties de celui-ci avec l'indicateur coloré ; et
c) détection d'un changement de couleur de l'indicateur coloré ; **caractérisé en ce que** la mise en contact du matériau (120) ou
de parties de celui-ci avec l'indicateur coloré comprend le chauffage du matériau (120) ou de parties de celui-ci dans l'ampoule transparente (100) sous vide et constituée au moins en partie de verre de borosilicate.

10. Procédé selon la revendication 9, dans lequel le chauffage comprend un chauffage par induction.

11. Procédé selon l'une des revendications 9 ou 10, dans lequel la détection est effectuée en utilisant une échelle de comparaison des couleurs, un capteur optique, un capteur chimique ou deux ou plusieurs de ces éléments.

12. Procédé selon l'une des revendications 9 à 11, comprenant en outre une étape, après la détection, consistant à comparer les informations obtenues dans le cadre de la détection avec les données stockées dans une base de données.
